(19) Europäisches Patentamt / European Patent Office / Office européen des brevets

(11) **EP 4 603 012 A1**

(12) **EUROPEAN PATENT APPLICATION**

(43) Date of publication:
**20.08.2025 Bulletin 2025/34**

(21) Application number: **24212460.0**

(22) Date of filing: **12.11.2024**

(51) International Patent Classification (IPC):
**A61B 5/06** $^{(2006.01)}$     **A61B 5/00** $^{(2006.01)}$
**A61M 25/00** $^{(2006.01)}$     **G06T 11/20** $^{(2006.01)}$

(52) Cooperative Patent Classification (CPC):
**A61B 5/6858; A61B 5/06; A61B 5/6859;**
**A61M 25/00; G06T 11/203**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **14.02.2024 US 202418441208**

(71) Applicant: **Biosense Webster (Israel) Ltd.**
**2066717 Yokneam (IL)**

(72) Inventor: **GLINER, Vadim**
**2066717 Yokneam (IL)**

(74) Representative: **Carpmaels & Ransford LLP**
**One Southampton Row**
**London WC1B 5HA (GB)**

(54) **METHODS FOR REAL TIME TRACKING OF A CATHETER SPLINE**

(57)     A method for real-time tracking of spline shape in a distal end assembly of a catheter. The method comprises: (a) receiving a first endpoint location data and a second endpoint location data based on a first and second position sensor assembly respectively mounted near a first and a second end of a flexible spline at a distal tip of a catheter; (b) performing a coarse determination of a location of an external control point of a quadratic Bezier curve for approximating a shape of the flexible spline, (c) refining the location of the control point; and (d) rendering a shape of the flexible spline based on the quadratic Bezier curve defined by the first endpoint location, the refined external control point location, and the second endpoint location.

FIG. 2

FIG. 3

**Description**

**TECHNOLOGICAL FIELD**

The presently disclosed subject matter generally relates to the field of real-time monitoring of a shape of a deflectable spline of a distal end assembly of a catheter.

**BACKGROUND**

A technique for estimating a curvature of a spline in a catheter end assembly using Bezier curves was described in U.S. Provisional Patent Application Number 17/874,224.

The technique includes receiving endpoint location data and spline tangent data from a pair of position sensors mounted on opposite ends of a distal tip of a catheter, wherein said distal tip includes a plurality of flexible splines and a plurality of electrodes disposed on each of the flexible splines and wherein said endpoint location data and spline tangent data is received while said distal tip is positioned within a heart chamber; based on said endpoint location data and spline tangent data, calculating Bezier curve control points; based on the Bezier curve control points, determining estimated positions of said plurality of electrodes; and updating an electro-anatomical map of said heart chamber that is rendered on a display based on the estimated positions determined.

A method of estimating a length of a Bezier curve is described in an article titled "How long is that Bézier" by Raph Levien, which can be found on the World Wide Web at "https://raphlinus.github.io/curves/2018/12/28/bezier-arclength.html".

**OVERVIEW**

Computing the length of a Bezier curve using the technique described in U.S. Patent Application Number 17/874,224 over several iterations may be relatively computation-intensive. Such a computation load may make it difficult and/or costly to render and display the spline curve of a catheter end device in real time, and even more so displaying several, for example 8 or 10, spline curves of a catheter end device in real time.

Some less computation-intensive methods of calculating a shape of a quadratic Bezier curve and determining estimated positions of electrodes on a spline in a catheter end device, are described below.

A length of a spline in a catheter end device is known - this is a physical attribute of the catheter end device. Locations of a distal end and a proximal end of the spline can also be known - they can be measured by various ways.

In one non-limiting example method, in order to draw a quadratic Bezier curve corresponding to a shape of a spline of a catheter end device, one can estimate a location of an external control point $P_c$ of the quadratic Bezier curve. Once such an estimated location for the quadratic Bezier curve control point that does not lie on the Bezier curve is known, as well as the measured locations of the distal end and the proximal end of the spline, a quadratic Bezier curve approximating the shape of the spline may be drawn, for example on a display.

In the present application and claims, in referring to a quadratic Bezier curve, we shall refer to the three control points of the quadratic Bezier curve as two end points of the quadratic Bezier curve and one external control point of the quadratic Bezier curve that does not lie on the Bezier curve.

A non-limiting example method disclosed herein uses the known length of the spline to determine an estimated location of the external control point based on the locations of the spline proximal and distal ends for which the length of the quadratic Bezier curve is equal to (or achieves a predefined degree of conformity with) the known length of the spline.

A non-limiting example of estimating an initial location of the external control point of the quadratic Bezier curve may comprise constraining the location of the quadratic Bezier curve to a first predetermined area based on the known mechanics of the distal end assembly. For example, estimating the location of the external control point of the quadratic Bezier curve may comprise determining that the initial location should be somewhere (i.e. constrained) on a specific line normal to and crossing at a mid-point of a straight line connecting the two end point locations of the quadratic Bezier curve.

Another non-limiting example of estimating the initial location of the external control point of the quadratic Bezier curve comprise determining that the initial location should be somewhere (i.e. constrained) on a specific line normal to the straight line connecting the two end point locations of the quadratic Bezier curve.

Another non-limiting example of estimating the initial location of the external control point of the quadratic Bezier curve may comprise determining that the initial location should be somewhere on a specific (pre-defined) path in relation to the two end point locations of the quadratic Bezier curve. The pre-defined path may be determined by analyzing mechanical attributes (e.g. shape, flexibility, structural integrity, etc.) of a distal end assembly of a catheter in a calibration facility and/or the type of medical indication of said catheter.

Another non-limiting example of estimating the initial location of the external control point of the quadratic Bezier curve comprises determining that the initial location should be somewhere in a specific area in relation to the two end point locations of the quadratic Bezier curve. The specific area (i.e. zone) may for example be determined based on known attributes of the distal end assembly of the catheter in a calibration facility such as mechanical properties of the deflectable splines (e.g. shape, flexibility, structural integrity, etc.) and/or the type of medical indication of said catheter.

In some examples, an iterative process is used whereby the length of the quadratic Bezier curve that is defined based on the initial location of the external control point of the quadratic Bezier curve and end point locations is determined based on integrating over the defined quadratic Bezier curve, the length of the quadratic Bezier curve is compared to the known length of the spline, and a new estimated location of the external control point of the quadratic Bezier curve is shifted until the estimated length of the quadratic Bezier curve is close enough to the known length of the spline. The shifting of the external control point of the quadratic Bezier curve may be constrained to a first predefined area (i.e. the specific area mentioned above). In some examples, a threshold is set to determine what difference between the estimated length of the quadratic Bezier curve and the known length of the spline is acceptable.

For example, the refining may include accurately calculating the length of the Bezier curve based on the initial location of the external control point, and, when the accurate (i.e. more-exact/precise) length of the quadratic Bezier curve is compared to the known length of the spline, a refined location for the quadratic Bezier curve may be identified.

By way of a non-limiting example the more-exact method for calculating the length of the quadratic Bezier curve may optionally be a method such as described in above-mentioned U.S. Patent Application Number 17/874,224. In the present disclosure, the method used for accurately computing the refined length of the quadratic Bezier curve may be generally referred to in the following as a numerical method (or numerical integration). It may for example include methods such as trapezoidal rule, Simpson's rule, quadrature methods, composite methods, etc.

In some examples, an iterative process is used whereby the length of the quadratic Bezier curve is calculated based on integrating over the defined Bezier curve. The length of the quadratic Bezier curve as computed based on integration is compared to the known length of the spline, and a location of the external control point of the quadratic Bezier curve is shifted until the calculated length of the quadratic Bezier curve is close enough to the known length of the spline. The shifting of the external control point of the quadratic Bezier curve may be constrained to a second predefined area. The first and second predefined area may of different shape. In some examples, a threshold is set to determine what difference between the calculated length of the quadratic Bezier curve and the known length of the spline is acceptable.

In some examples, shapes of a plurality of splines may be calculated using the above method(s) - the more splines for which such shapes are calculated using a simplified method for initial estimation, the greater the saving in computational load.

## BRIEF DESCRIPTION OF THE DRAWINGS

In order to better understand the subject matter that is disclosed herein and to exemplify how it may be carried out in practice, examples will now be described, by way of non-limiting example only, with reference to the accompanying drawings, in which:

Figure 1 illustrates an exemplary system in which one or more features of the disclosure subject matter can be implemented according to one or more examples;

Figure 2 illustrates a catheter end assembly, according to an example;

Figure 3 is an illustration of a simplified method to estimate a length of a quadratic Bezier curve based on the length of a chord connecting the first and second endpoint locations and the length of a control polyline formed by the first endpoint location, the external control point location, and the second endpoint location according to some examples;

Figure 4A is a simplified illustration of an external control point of the quadratic Bezier curve located on a line according to an example;

Figure 4B is a simplified illustration of an external control point of the quadratic Bezier curve located on a specific linear path according to an example;

Figure 5A is a simplified flow chart illustration of a method for real-time tracking of spline shape in a distal end assembly of a catheter according to an example; and

Figure 5B is a simplified flow chart illustration of a method for real-time tracking of spline shape in a distal end assembly of a catheter according to an example.

## DETAILED DESCRIPTION

The presently disclosed subject matter generally relates to the field of computing a shape of a quadratic Bezier curve, and more specifically to a rapid method for finding a location of an external control point for computing a shape of a quadratic Bezier curve representing a shape of a spline in a catheter end assembly and even more specifically, but not exclusively, to visualization of a catheter end assembly using rapid calculation of shape(s) of Bezier curve(s).

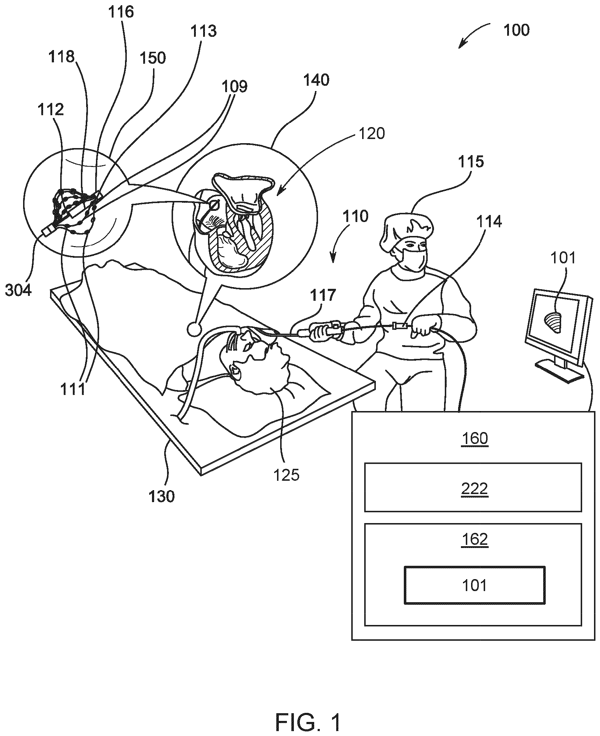

Reference is made to Figure 1 which illustrates an exemplary system in which one or more features of the disclosure subject matter can be implemented according to one or more examples.

Figure 1 is a diagram of an example system (e.g., medical device equipment), shown as a system 100, in which one or more features of the subject matter herein can be implemented according to one or more examples. All or part of the system 100 can be used to detect, diagnose, and/or treat cardiac conditions.

The system 100, as illustrated, includes a catheter 110, which includes a manipulator 114, a shaft 112 disposed through a sheath 113, and a distal tip in the shape of a basket (referred to herein as a "catheter basket" 116). The catheter basket 116 includes a plurality of electrodes 111 disposed on a plurality of bendable splines 109. A puller element 118 pulls or pushes the distal end 304 of the catheter basket 116, expanding or collapsing the catheter basket 116. In some examples, the catheter basket 116 includes one or more position sensors that allow sensing of the position of the proximal end and distal end of the catheter basket 116.

Also illustrated in Figure 1 are a physician 115 (or a medical professional, technician, clinician, etc.), a heart 120, a patient 125, and a bed 130 (or a table). Note that insets 140 and 150 show the heart 120 and the catheter 110 in greater detail. The system 100 also, as illustrated, includes a console 160 (including one or more processors 222 and memories 162 providing controlling and processing capability) and a display 165. Note further each element and/or item of the system 100 is representative of one or more of that element and/or that item. The example of the system 100 shown in Figure 1 can be modified to implement the embodiments disclosed herein. The disclosed embodiments can similarly be applied using other system components and settings. Additionally, the system 100 can include additional components, such as elements for sensing electrical activity, wired or wireless connectors, processing and display devices, or the like.

Treatment for cardiac conditions such as cardiac arrhythmia often requires obtaining a detailed mapping of cardiac tissue, chambers, veins, arteries and/or electrical pathways. For example, a prerequisite for performing a catheter ablation (as described herein) successfully is that the cause of the cardiac arrhythmia is accurately located in a chamber of the heart 120. Such locating may be done via an electrophysiological investigation during which electrical potentials are detected and spatially resolved with a mapping catheter (e.g., the catheter 110) introduced into the chamber of the heart 120. This electrophysiological investigation, the so-called electro-anatomical mapping, thus provides 3D mapping data which can be displayed on a monitor. In many cases, the mapping function and a treatment function (e.g., ablation) are provided by a single catheter or group of catheters such that the mapping catheter also operates as a treatment (e.g., ablation) catheter at the same time. Mapping software 101 interfaces with catheter 110 to perform the mapping operations as described in further detail herein.

The catheter 110 includes a plurality of flexible splines 109 and a plurality of electrodes 111 disposed on each of the flexible splines 109. The catheter 110 is configured to obtain biometric data, such as electrical signals of an intra-body organ (e.g., the heart 120), and/or to ablate tissue areas of thereof (e.g., a cardiac chamber of the heart 120).

In some examples the catheter 110 is a basket catheter. The basket catheter can be designed such that when deployed into a patient's body, its electrodes can be held in intimate contact against an endocardial surface. As an example, a basket catheter can be inserted into a lumen, such as a pulmonary vein ("PV"). The basket catheter can be inserted into the PV with a proximal end at maximal distance from distal end, such that the basket catheter does not occupy its maximum volume while being inserted into the PV. The basket catheter can expand by moving proximal end towards distal end while inside the PV, such that those electrodes on the basket catheter are in contact with an entire circular section of the PV. Such contact with an entire circular section of the PV, or any other lumen, can enable efficient imaging and/or ablation.

The catheter 110 and other items of the system 100 may be connected to the console 160. The console 160 can include any computing device, which employs the mapping software 101. According to an exemplary embodiment, the console 160 includes the one or more processors 222 (any computing hardware) and the memory 162 (any non-transitory tangible media), where the one or more processors 222 execute computer instructions with respect to the mapping software 101 and the memory 162 stores these instructions for execution by the one or more processors 222. For instance, the console 160 can be configured to receive and/or store the biometric data on a database of the memory 162, process the biometric data, and determine if a given tissue area conducts electricity.

The display 165, which can be any electronic device for the visual presentation of the biometric data, is connected to the console 160. According to an exemplary embodiment, during a procedure, the console 160 can facilitate on the display 165 a presentation of a body part rendering to the physician 115 and store data representing the body part rendering in the memory 162. For instance, maps depicting motion characteristics can be rendered/constructed based on the trajectory information sampled at a sufficient number of points in the heart 120.

Among other things, the mapping software 101 maps position of the electrodes 111 of the catheter 110. The catheter 110 has position tracking elements on proximal and distal ends of the catheter basket 116, but the electrodes 111 themselves may not have associated position sensors. The electrodes 111 may be disposed on flexible splines 109 which bend based both on relative positions of the proximal and distal ends of the catheter basket 116 as well as deflection of the entire catheter 110 with respect to the shaft 112. Thus, the mapping software 101 performs operations to determine the shape and positions of the splines 109 based on the detected positions and orientations of the proximal and distal ends of the catheter basket 116 as well as the deflection angle of the catheter basket 116.

In some embodiments the mapping software 101 generates Bezier curves to represent the shape, position, and/or deflection of the splines 109 and obtains positions of the electrodes 111 based on the shape, position, and/or deflection.

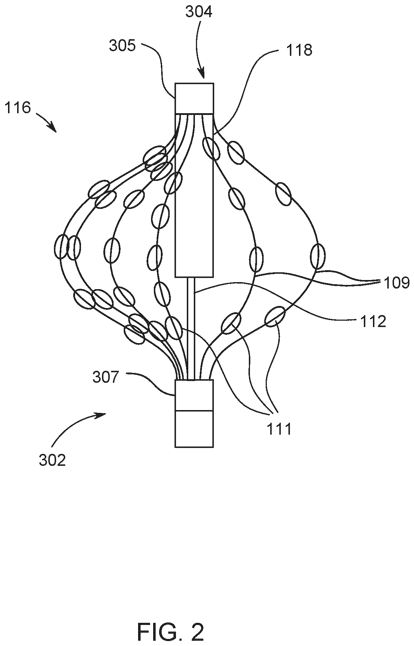

Reference is now made to Figure 2, which illustrates a catheter end assembly, according to an example.

Figure 2 illustrates a catheter end assembly in a shape of a basket 116, which includes a shaft 112 coupled to a proximal end 302 of the catheter basket 116 and to a distal end 304 of the catheter basket 116. Splines 109 are coupled to a proximal end 302 and a distal end 304 of the catheter basket 116. Electrodes 111 are disposed on the splines 109. The proximal end 302 and distal end 304 are movable with respect to each other. A puller element 118, which is coupled to the distal end 304 of the catheter basket 116, can be pulled towards the proximal end 302 of the catheter basket to move the distal end 304 closer to the proximal end 302, or can be pushed from the proximal end 302 to move the distal end 304 farther from the proximal end 302.

Moving the distal end 304 with respect to the proximal end 302 causes the splines 109 to deform. In addition, it is possible for the catheter basket 116 to be deflected at an angle with respect to the shaft 112 at the proximal end 302. In other words, it is possible for the basket assembly to bend at the proximal end 302, with respect to an "incoming angle" of the shaft 112.

For example, when a part of the catheter basket 116 such as one or more of the splines 109 contacts anatomical structure, the catheter basket 116 deflects at an angle with respect to the shaft 112 at the proximal end 302. The shape of the splines 109 and thus the positions of the electrodes 111 thus depends on the relative positions of the proximal end 302 and the distal end 304, as well as the angle of deflection of the catheter 110 with respect to the shaft 112 at the proximal end 302.

The proximal end 302 and distal end 304 may each include one or more position sensors (the proximal end position sensor 307 and the distal end position sensor 305), so that mapping software 101 is able to determine their three-dimensional ("3D") positions directly (e.g., in conjunction with the position system(s) 223). However, some or even all of the splines 109 may not have position sensors and, moreover, can change shape. For this reason, mapping software 101 estimates the positions and shapes of the splines 109 in order to use that information for calculating shapes of the splines and optionally displaying the splines 109.

In some examples 3 single axis sensors may be located at the distal end 304.

In some examples not all splines have a sensor.

In some examples a distal end of the end assembly is rigid and can define a plane. Based on that it is possible to determine an endpoint of each spline.

Introduction

One approach to computing curve arc length, including quadratic Bezier curve length, is to sample the curve at a sequence of points, then add up the lengths of all segments between consecutive points. Such a method is equivalent to flattening a curve into lines and adding up all the line lengths. The method is simple and robust, but the method is relatively slow, or computationally expensive, when high accuracy is desired. The accuracy quadruples with every doubling of the number of samples. Another way of putting it, the number of samples grows as $O(\sqrt{N})$, where N is a reciprocal of error tolerance.

A rapid method of estimating a length of a quadratic Bezier curve potentially enables rapidly determining a shape of a spline in a catheter end assembly, and based on that, estimated positions of electrodes on the spline based on knowing their location along the spline. Such a method also enables updating the shape of the spline and visualizing a device at a distal end of a catheter in real time. Such a method potentially enables updating an electro-anatomical map of a catheter end assembly and a heart chamber that is rendered on a display.

By way of a non-limiting example, a simple, rapid method of estimating an initial location for an external control point of a quadratic Bezier curve may involve using an analytic formula for estimating a length of the quadratic Bezier curve based on a length of a chord connecting a first and second endpoint locations and a length of a control polyline formed by the first endpoint location, the external control point location, and the second endpoint location. The initial location of the external control point may be determined as the (or one of the) location(s) for which the estimated length of the quadratic Bezier curve equals the known spline length. The determination of the initial location of the control point may also involve constraining the location of the control point to a specific path.

The term control polyline may be used in the present specification and claims to refer to a line formed by connecting a first line which connects a first Bezier curve endpoint with the external control point, and a second line which connects the external control point to the second Bezier curve endpoint. The control polyline is also termed a Bézier polygon or a control polygon. However, in order not to confuse the above-mentioned connected lines with a closed polygon, the term polyline is used herein.

The estimate for the length of the quadratic Bezier curve proposed in Raph Levien's article can approximate the actual length of the quadratic Bezier curve with an error of +/-2%.

In some examples, estimating the initial location of the external control point of the quadratic Bezier curve may provide an external control point for the quadratic Bezier curve which may serve as a starting point for more accurately identifying the external control point for which the corresponding quadratic Bezier curve matches the known length of the catheter spline. The accurate identification of said external control point (i.e. refining the location of the control point) may comprise calculating a length of the quadratic Bezier curve for the initial location, comparing the calculated length to the actual length of the spline, and determining whether the lengths are close enough for rendering a shape of the spline using a shape of the quadratic Bezier curve or if the control point location needs adjustment. In some examples, refining the location involves shifting or adjusting the location of the external control point to minimize a difference between the calculated length of the quadratic Bezier curve and the actual length of the catheter spline to be below a specific threshold.

In some examples, estimating the initial location of the external control point of the quadratic Bezier curve may provide an external control point for the quadratic Bezier curve which may serve as a starting point for iteratively determining a more accurate location of the external control point by comparing the calculated length to the actual length of the spline, and determining whether the lengths are close enough for rendering a shape of the spline using a shape of the quadratic Bezier curve. Otherwise, the estimated location of the external control point is shifted and the calculation is repeated.

In some examples, estimating the location of initial location of the quadratic Bezier curve may provide an initial location for a control point for the quadratic Bezier curve which may already be useful for rendering a shape of the spline based on the quadratic Bezier curve.

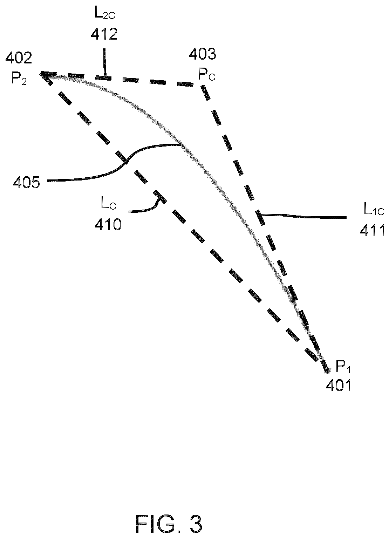

Reference is now made to Figure 3, which is an illustration of a simplified method to estimate a length of a quadratic Bezier curve based on the length of a chord connecting the first and second endpoint locations and the length of a control polyline formed by the first endpoint location, the external control point location, and the second endpoint location according to some examples.

Figure 3 shows a quadratic Bezier curve 405, extending from a first endpoint P1 401 to a second end point P2 402, and a quadratic Bezier curve control point Pc 403.

The quadratic Bezier curve 405 is a path traced by a function B(t), given points $P_1$ 401, quadratic Bezier curve control point Pc 403, and $P_2$ 402, where:

$$B(t) = P_c + (1-t)^2(P_1 - P_c) + t^2(P_2 - P_c) \quad \text{where } 0 \leq t \leq 1 \qquad \text{Equation 1}$$

Taking the end points $P_1$ 401 and $P_2$ 402 to be known locations of end points of a spline of a catheter end assembly and selecting an initial external control point of the quadratic Bezier curve Pc 403 for the curve 405 the length of the quadratic Bezier curve 405 can be estimated.

$L_p$, a control polyline ($L_{1c}+L_{2c}$) of the quadratic Bezier curve, over-estimates a length $L_s$ of the quadratic Bezier curve 405.

A length of a straight-line or chord $L_c$ 410 between $P_1$ 401 and $P_2$ 402 under-estimates the length of the curve 405.

In some examples, an analytic formula for an estimate $L_{est}$ of the length $L_s$ of the curve 405 may be given by:

$$L_{est} = (2L_c + L_p)/3 = 1.002L_s \qquad \text{Equation 2}$$

It is noted that the estimated length $L_{est}$ calculated is within approximately 0.2 percent of the actual length $L_s$ of the quadratic Bezier curve 405.

It is noted that when the shape of the quadratic Bezier curve 405 is taken to represent a shape of a catheter end assembly spline 109, the estimated length $L_{est}$ calculated may be considered to be within approximately 0.2 percent of the actual length of the catheter end assembly spline 109.

We now describe methods of using an example calculation such as the example calculation of Equation 2, not for calculating the length $L_{est}$, but for estimating a location of an external control point $P_c$ 403 knowing the length of the spline.

Locations of end points

In some examples, locations of the end points $P_1$ 401 and $P_2$ 402 are selected based on known locations of ends of the catheter end assembly, and a geometric relation between the known locations of ends of the catheter end assembly and end points of the spline 109.

In some examples, locations of the end point(s) $P_1$ 401 and/or $P_2$ 402 are selected based on known locations of ends of the spline, obtained for example by imaging the catheter end assembly within a patient's body.

In some examples, the locations of the end point(s) $P_1$ 401 and/or $P_2$ 402 are determined based on a location sensor placed at each end of the spline 109.

Examples of coarse determination of a location of an external control point by direct calculation

A non-limiting example of estimating the initial location of the external control point of the quadratic Bezier curve comprises determining that the initial location should be somewhere on a specific line normal to and crossing at a mid-point of a straight line connecting the two end point locations (i.e. the chord) of the quadratic Bezier curve.

Other non-limiting examples of estimating the initial location of the external control point of the quadratic Bezier curve may include determining that the initial location should be somewhere on a specific path, possibly a linear path, or area relative to the two end point locations of the quadratic Bezier curve.

Another non-limiting example of estimating the initial location of the external control point of the quadratic Bezier curve is described by referring again to Figure 3. An initial location of the external control point of the quadratic Bezier curve $P_c$ 403 may be determined based on data indicative of a direction of the catheter spline near the first and second endpoints. Sensors located at or near the ends of the spline may allow estimating the direction of the catheter spline near the first and second endpoints. Thus, determining the initial location may be derived from a triangle, e.g., polygon, defined by $P_1$ 401 $P_2$ 402 and $P_c$ 403 as follows: the locations of two points, $P_1$ 401 and $P_2$ 402 are known, so the line $L_c$ 410 is defined. The angles of the line $L_{1c}$ at $P_1$ 401 and of the line $L_{2c}$ at $P_2$ 402 may be determined based upon data collected by said sensors.

When the line $L_c$ 410 and the angles at $P_1$ 401 and $P_2$ 402 are all known, the triangle is fully defined and the location of the external control point $P_c$ 403 can be calculated.

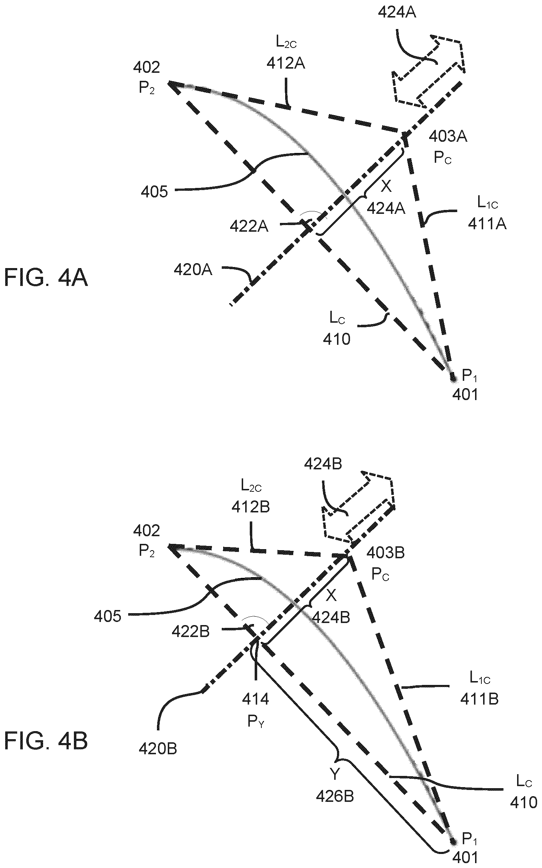

Reference is now made to Figure 4A, which is a simplified illustration of an external control point of the quadratic Bezier curve located on a line according to an example.

Figure 4A shows a quadratic Bezier curve 405, extending from a first endpoint $P_1$ 401 to a second end point $P_2$ 402, and an external control point $P_c$ 403A.

Figure 4A also shows a line 420A perpendicular (see angle 422A) to the line $L_c$ 410 connecting the first endpoint $P_1$ 401 to the second end point $P_2$ 402, crossing the line $L_c$ 410 at a midpoint between the two endpoints $P_1$ 401 and $P_2$ 402.

In some examples, a location of the external control point $P_c$ 403A is constrained to be on a specific path such as the line 420A. A coarse determination of the location of the external control point $P_c$ 403 may be analytically calculated as follows: the lengths of $L_{1c}$ 411A and $L_{2c}$ 412A are equal, so by setting the estimated length $L_{est}$ to be equal to the length of the spline $L_s$, Equation 2 enables calculating their lengths as follows:

$$L_s = (2L_c + L_p)/3 = (2L_c + L_{1c} + L_{2c})/3 = (2L_c + 2L_{1c})/3 \qquad \text{Equation 3}$$

Locations of $P_1$ 401 and $P_2$ 402 are known, so the line $L_c$ 410 is defined. The (equal) lengths of the lines $L_{1c}$ 411 and $L_{2c}$ 412 are calculated by Equation 3, so the location of the external control point $P_c$ 403 can be calculated.

X 424A denoting a distance between $P_c$ 403A and the midpoint of the line $L_c$ 410, X 424A defines an example location of the external control point $P_c$ 403 as measured from the midpoint between known locations $P_1$ 401 and $P_2$ 402 in a perpendicular direction from the line $L_c$ 410. Equation 4 below enables direct estimation of the location of the external control point $P_c$ 403:

$$X = \sqrt{(\tfrac{3}{2}L_s - L_c)^2 - (\tfrac{1}{2}L_c)^2} \qquad \text{Equation 4}$$

Reference is now made to Figure 4B, which is a simplified illustration of an external control point of the quadratic Bezier curve located on a specific linear path according to an example.

Figure 4B shows a quadratic Bezier curve 405, extending from a first endpoint $P_1$ 401 to a second end point $P_2$ 402, and an external control point $P_c$ 403B.

Figure 4B also shows a line 420B perpendicular (see angle 422B) to the line $L_c$ 410 connecting the first endpoint $P_1$ 401 to the second end point $P_2$ 402, crossing the line $L_c$ 410 at some known point $P_Y$ 414 between the two endpoints $P_1$ 401 and $P_2$ 402.

In some examples, a location of the external control point $P_c$ 403B is selected to be on the line 420B. The location of the external control point $P_c$ 403 may also be calculated by considering a first triangle defined by $P_1$ 401 $P_Y$ 414 and $P_c$ 403B, and a second triangle defined by P2 402 $P_Y$ 414 and Pc 403B as follows: The distance between $P_1$ 401 and $P_Y$ 414 is known, and now defined as Y 426B, the distance between $P_2$ 402 and $P_Y$ 414 is also known and is equal to (Lc - Y).

The distance of Pc 403B from $P_Y$ 414 is defined as X 424B.

Since $L_s$, $L_c$ (the distance between P1 401 and P2 402) and Y 426B are known, and the angle 422B is a right angle, X 424B can be calculated.

Determination of a location of an external control point by calibration

In some examples a catheter end assembly may be measured in a calibration lab and a shape of the spline(s) can be measured under various conditions and various contortions of the catheter end assembly. Under such conditions locations for the external control point $P_c$ for drawing correct shapes of the spline(s) can be determined and recorded, so that inputs of spline end-point locations and directions of direction sensor(s) such as single-axis-sensors can be used to determine suitable locations for the external control point $P_c$ based on the recorded data. For example, Look-Up-Table may be used.

Examples of external control point location determination

In some of the examples the external control point Pc 403 may be selected (i.e. constrained) to be on a specific linear path (see line 420B of Figure 4B) which passes the line $L_c$ 410 connecting the end points of the quadratic Bezier curve 405 at a 90 degree angle. See for example the description of Figures 4A and 4B.

In some of the examples the external control point Pc 403 may be selected (i.e. constrained) to be on a specific linear path (see line 420A of Figure 4A) which is perpendicular to, and passes at a midpoint of, the first line $L_c$ 410 connecting the end points of the quadratic Bezier curve 405. See for example the description of Figure 4A.

In some of the examples the external control point Pc 403 may be selected (i.e. constrained) to be along a specific linear path at some angle to the straight-line 410 between $P_1$ 401 and $P_2$ 402 which crosses the line 410 at some location between $P_1$ 401 and $P_2$ 402.

In some examples the location of the external control point Pc 403 relative to the end points $P_1$ 401 and $P_2$ 402 may be constrained within a specific path or area dependent on known geometrical and/or mechanical properties of the catheter spline and/or its medical indication.

In some examples, determining the location of the external control point Pc 403 relative to the end points $P_1$ 401 and $P_2$ 402 may involve measuring and/or analyzing a shape of the spline as part of a catheter end assembly measured/analyzed outside of a patient's body.

Optionally increasing accuracy using a second method to calculate length

In some examples, after a candidate location for the control point is identified in the coarse determination stage (e.g. after one or more iterations using an estimation formula bring the level of accuracy under a desired first threshold), a more accurate and computationally more intensive method is optionally used to further increase the accuracy.

In some examples the length of the quadratic Bezier curve may optionally be computed by a numerical method along the Bezier curve from one end point of the Bezier curve to the other end of the Bezier curve. If the quadratic Bezier curve length as calculated by said numerical method is not close enough to the actual spline length, the control point may be shifted (optionally within the second specific path/area) and a quadratic Bezier curve length may be calculated again and compared to the actual spline length, until it is close enough.

In some examples the length of the quadratic Bezier curve may optionally be computed, by way of a non-limiting example, by a method such as described in U.S. Provisional Patent Application Number 17/874,224. If the quadratic Bezier curve length as calculated by numerical method is not close enough to the actual spline length, the control point may be shifted (optionally within the second specific path/area) and a quadratic Bezier curve length may be calculated again and compared to the actual spline length, until it is close enough.

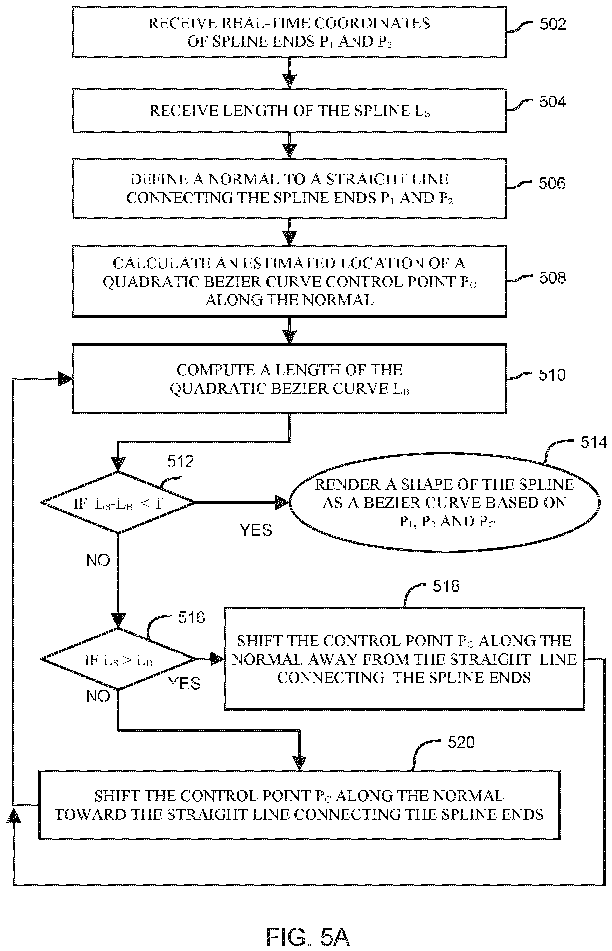

Reference is now made to Figure 5A, which is a simplified flow chart illustration of a method for real-time tracking of spline shape in a distal end assembly of a catheter according to an example.

In a first step 502, the method includes receiving a first endpoint location data $P_1$ based on a first position sensor assembly mounted near a first end of a flexible spline at a distal tip of a catheter and receiving a second endpoint location data $P_2$ based on a second position sensor assembly mounted near a second end of the flexible spline.

In a second step 504, the method includes receiving a known length of the flexible spline $L_s$. In a third step 506, the method includes defining a normal to a straight line connecting the spline end locations $P_1$ and $P_2$ (i.e., the chord). In a fourth step 508, the method includes calculating an estimated (coarse) location of an external control point $P_c$. The coarse determination may be based on a relationship between a known length of the flexible spline and an estimated length of the quadratic Bezier curve. The estimated length of the quadratic Bezier curve may be computed based on the length of a chord connecting the first and second endpoint locations and the length of a control polyline formed by the first endpoint location, the control point location, and the second endpoint location. The coarse location of the control point may be a location on the normal to the chord (i.e., the first predefined path/area) for which the estimated length of the quadratic Bezier curve is equal (or sufficiently close) to the known length of the spline.

In a fifth step 510, the method includes computing a length of the quadratic Bezier curve $L_B$ for the coarse location of the control point $P_c$, for example using a more accurate method such as numerical integration.

In a sixth step 512, the method includes comparing $L_B$ and $L_S$.

In a seventh step 514, if an absolute value $|L_s - L_B| < T$, where T is a threshold value, then the quadratic Bezier curve $L_B$ defined by the end point locations $P_1$ and $P_2$ and the external control point $P_c$ is close enough to the desired spline shape and the method includes rendering a shape of the spline as a Bezier curve based on $P_1$, $P_2$ and $P_c$ (514)

In an eighth step 516, if the absolute value $|L_s - L_B| > T$, the method includes comparing real values of $L_s$ and $L_B$.

In a ninth step 518, if $L_s > L_B$ (516), then the quadratic Bezier curve $L_B$ defined by the end point locations $P_1$ and $P_2$ and the external control point $P_c$ is *not* yet close enough to the desired spline shape and the method includes shifting the control point Pc along the normal *away from* the straight line connecting the spline ends (518) and returning to computing a length of the quadratic Bezier curve $L_B$ (510) according to the fifth step.

In a tenth step, if $L_s < L_B$, the method includes shifting the control point $P_c$ along the normal *toward* the straight line connecting the spline ends (520) and returning to computing a length of the quadratic Bezier curve $L_B$ (510) according to the fifth step.

In some cases, the endpoint location data $P_1$ and $P_2$ may be determined based on magnetic position sensing.

In some cases, the endpoint location data $P_1$ and $P_2$ may be determined based on imaging the catheter end assembly.

In some cases, the length of the flexible spline $L_s$ may optionally be stored in and/or retrieved from memory.

In some cases, the method of Figure 5A includes using Equation 4 for calculating the estimated location of the external control point $P_c$ in step 508 where Ls is the known length of the flexible spline and Lc is the calculated distance between the first endpoint location data $P_1$ and the second endpoint location data $P_2$.

In some examples, the normal may cross the line between the two end points at the midpoint between the two end points.

In some examples the normal may cross the line between the two end points at other locations, optionally based on known mechanics of the catheter end assembly.

In some examples the location at which the normal crosses the line between the two end points may be predefined and/or stored in memory.

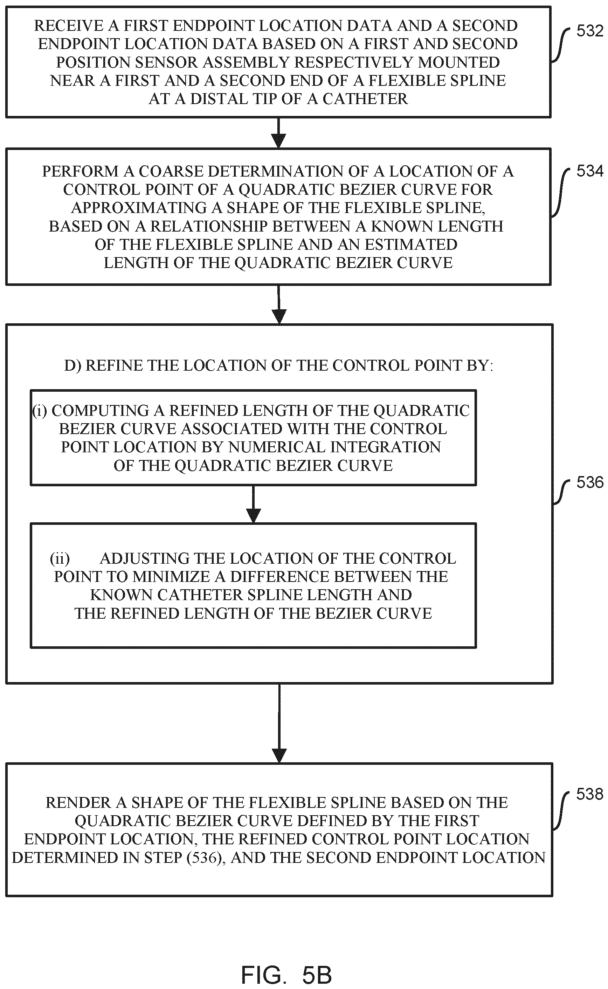

Reference is now made to Figure 5B, which is a simplified flow chart illustration of a method for real-time tracking of spline shape in a distal end assembly of a catheter according to an example.

In a first step 532, the method includes receiving a first endpoint location data and a second endpoint location data based on a first and second position sensor assembly respectively mounted near a first and a second end of a flexible spline at a distal tip of a catheter.

In a second step 534, the method includes performing a coarse determination of a location of an external control point of a quadratic Bezier curve for approximating a shape of the flexible spline, wherein the quadratic Bezier curve is defined by a first and second points corresponding to the first and second endpoints locations and by said external control point, said coarse determination being based on a relationship between a known length of the flexible spline and an estimated length of the quadratic Bezier curve.

In a third step 536, the method includes refining the location of the control point by:

(i) computing a refined length of the quadratic Bezier curve associated with the external control point location determined in the second step (534) by numerical integration of said quadratic Bezier curve; and

(ii) adjusting the location of the external control point to minimize a difference between the known catheter spline length and the refined length of the Bezier curve.

In a fourth step 538, the method includes rendering a shape of the flexible spline based on the quadratic Bezier curve defined by the first endpoint location, the refined control point location determined in step 536, and the second endpoint location.

In some embodiments the estimated length of the quadratic Bezier curve in the coarse determination is computed based on the length of a chord connecting the first and second endpoint locations and the length of a control polyline formed by the first endpoint location, the control point location, and the second endpoint location.

EXAMPLES

Following is a non-exclusive list of some exemplary examples of the disclosure. The present disclosure also includes examples which include fewer than all the features in an example and examples using features from multiple examples, even if not listed below.

Example 1:

A method for real-time tracking of spline shape in a distal end assembly of a catheter, including:

a) receiving (532) a first endpoint location data and a second endpoint location data based on a first and second position sensor assembly respectively mounted near a first and a second end of a flexible spline at a distal tip of a catheter,

b) performing a coarse determination (534) of a location of an external control point of a quadratic Bezier curve for approximating a shape of the flexible spline, wherein the quadratic Bezier curve is defined by a first and second points corresponding to the first and second endpoints locations and by the external control point, the coarse determination being based on a relationship between a known length of the flexible spline and an estimated length of the quadratic Bezier curve, wherein the estimated length of the quadratic Bezier curve in the coarse determination is computed based on the length of a chord connecting the first and second endpoint locations and the length of a control polyline formed by the first endpoint location, the control point location, and the second endpoint location;

c) refining the location of the control point (536) by

(i) computing a refined length of the quadratic Bezier curve associated with the external control point location determined by the coarse determination by numerical integration of the quadratic Bezier curve,

(ii) adjusting the location of the external control point to minimize a difference between the known catheter spline length and the refined length of the Bezier curve, and

d) rendering a shape of the flexible spline (538) based on the quadratic Bezier curve defined by the first endpoint location, the refined external control point location, and the second endpoint location.

Example 2:

The method of example 1, wherein the external control point location is constrained to a predefined path and the coarse determination includes determining the location of the external control point on the predefined path for which the estimated length of the quadratic Bezier curve is equal to the known length of the flexible spline.

Example 3:

The method of any of examples 1 or 2, wherein the coarse determination of the location of the external control point is computed using a formula $L_s = (2L_c+L_p)/3$ wherein $L_s$ is the known length of the spline, $L_c$ is the length of the chord connecting the first and second endpoint locations and $L_p$ is the length of a control polyline of the Bezier curve formed by the first endpoint location, the external control point location, and the second endpoint location.

Example 4:

The method of any of examples 2 or 3, wherein the predefined path is a linear path median and normal to the chord connecting the first and second endpoint locations.

Example 5:

The method of example 1, wherein performing a coarse determination includes:

(i) iteratively selecting a candidate location for the control point,
(ii) for each candidate location, computing an estimated length of the quadratic Bezier curve, wherein the estimated length is based on the length of a control polyline formed by the first endpoint location, the control point location, and the second endpoint location, and the length of a chord connecting the first and second endpoint locations,
(iii) comparing the estimated length of the quadratic Bezier curve with the known length of the catheter spline, and
(iv) identifying a candidate location for the control point for which a difference between the known catheter spline length and the estimated length of the Bezier curve is below a first threshold.

Example 6:

The method of example 5 wherein the estimated length of the quadratic Bezier curve is computed using a formula $L_{est} = (2L_c+L_p)/3$ wherein $L_{est}$ is the estimated length of the quadratic Bezier curve, $L_c$ is the length of the chord connecting the first and second endpoint locations and $L_p$ is the length of a control polyline formed by the first endpoint location, the control point location, and the second endpoint location.

Example 7:

The method of any of examples 1 to 6, wherein in the coarse determination, the candidate location is constrained to a first predefined area.

Example 8:

The method of any of examples 1 to 7, wherein in the refining of the external control point location, the location of the external control point is constrained to a second predefined area.

Example 9:

A system including a console configured to:

a) receive a first endpoint location data and a second endpoint location data based on a first and second position sensor assembly respectively mounted near a first and a second end of a flexible spline at a distal tip of a catheter,
b) perform a coarse determination of a location of an external control point of a quadratic Bezier curve for approximating a shape of the flexible spline, wherein the quadratic Bezier curve is defined by a first and second points corresponding to the first and second endpoints locations and by the external control point, the coarse determination being based on a relationship between a known length of the flexible spline and an estimated length of the quadratic Bezier curve, wherein the estimated length of the quadratic Bezier curve in the coarse determination is computed based on the length of a chord connecting the first and second endpoint locations and the length of a control polyline formed by the first endpoint location, the control point location, and the second endpoint location;
c) refine the location of the external control point by

(i) computing a refined length of the quadratic Bezier curve associated with the external control point location determined in step b) by numerical integration of the quadratic Bezier curve,
(ii) adjusting the location of the external control point to minimize a difference between the known catheter spline length and the refined length of the Bezier curve, and

d) render a shape of the flexible spline based on the quadratic Bezier curve defined by the first endpoint location, the refined external control point location determined in step c), and the second endpoint location.

Example 10.

A non-transitory computer-readable medium storing instructions that, when executed by a processor, cause the processor to perform operations including

a) receiving a first endpoint location data and a second endpoint location data based on a first and second position sensor assembly respectively mounted near a first and a second end of a flexible spline at a distal tip of a catheter,
b) performing a coarse determination of a location of an external control point of a quadratic Bezier curve for approximating a shape of the flexible spline, wherein the quadratic Bezier curve is defined by a first and second points corresponding to the first and second endpoints locations and by the external control point, the coarse determination being based on a relationship between a known length of the flexible spline and an estimated length of the quadratic Bezier curve,
c) refining the location of the external control point by

(i) computing a refined length of the quadratic Bezier curve associated with the external control point location determined in step b) by numerical integration of the quadratic Bezier curve,
(ii) adjusting the location of the external control point to minimize a difference between the known catheter spline length and the refined length of the Bezier curve, and

d) rendering a shape of the flexible spline based on the quadratic Bezier curve defined by the first endpoint location, the refined external control point location determined in step c), and the second endpoint location.

Unless specifically stated otherwise, as apparent from the present disclosure, it is appreciated that throughout the specification discussions utilizing terms such as "processing", "computing", "comparing", "determining", "estimating" or the like, refer to the action(s) and/or process(es) of a computer that manipulate and/or transform data into other data, said data represented as physical, such as electronic, quantities and/or said data representing the physical objects. The term "computer" should be expansively construed to cover any kind of hardware-based electronic device with data processing capabilities.

The various illustrative logical blocks, modules, and algorithm steps described in connection with the examples disclosed herein can be implemented as electronic hardware, computer software, or combinations of both. To clearly illustrate this interchangeability of hardware and software, various illustrative components, blocks, modules, and steps have been described above generally in terms of their functionality. Whether such functionality is implemented as hardware or software depends upon the particular application and design constraints imposed on the overall system. The described functionality can be implemented in varying ways for each particular application, but such implementation decisions should not be interpreted as causing any departure from the scope of the disclosure.

It will also be understood that a system according to the present disclosure may be, at least partly, implemented on a suitably programmed computer. Likewise, the present disclosure contemplates a computer program being readable by a computer for executing the method of the present disclosure. The present disclosure further contemplates a non-transitory computer-readable memory tangibly embodying a program of instructions executable by the computer for executing the method of the present disclosure.

**Claims**

1. A system comprising a console configured to:

   a) receive a first endpoint location data and a second endpoint location data based on a first and second position sensor assembly respectively mounted near a first and a second end of a flexible spline at a distal tip of a catheter;
   b) perform a coarse determination of a location of an external control point of a quadratic Bezier curve for approximating a shape of the flexible spline, wherein the quadratic Bezier curve is defined by a first and second points corresponding to the first and second endpoints locations and by said external control point, said coarse determination being based on a relationship between a known length of the flexible spline and an estimated length of the quadratic Bezier curve; wherein the estimated length of the quadratic Bezier curve is computed based on the length of a chord connecting the first and second endpoint locations and the length of a control polyline formed by the first endpoint location, the control point location, and the second endpoint location;
   c) refine the location of the external control point by:

      (i) computing a refined length of the quadratic Bezier curve associated with the external control point location determined in step b) by numerical integration of said quadratic Bezier curve;
      (ii) adjusting the location of the external control point to minimize a difference between the known catheter spline length and the refined length of the Bezier curve; and

   d) render a shape of the flexible spline based on the quadratic Bezier curve defined by the first endpoint location, the refined external control point location determined in step c), and the second endpoint location.

2. The system of claim 1, wherein the external control point location is constrained to a predefined path and performing the coarse determination comprises determining the location of the external control point on said predefined path for which the estimated length of the quadratic Bezier curve is equal to the known length of the flexible spline.

3. The system of claim 1 or claim 2, wherein the console is configured to perform the coarse determination of the location of the external control point by using a formula $L_s = (2L_c + L_p)/3$ wherein $L_s$ is the known length of the spline, $L_c$ is the length of the chord connecting the first and second endpoint locations and $L_p$ is the length of a control polyline of the Bezier curve formed by the first endpoint location, the external control point location, and the second endpoint location.

4. The system of claim 2 or claim 3, wherein the predefined path is a linear path median and normal to the chord connecting the first and second endpoint locations.

5. The system of any preceding claim, wherein in the coarse determination, the location of the external control point is constrained to a first predefined area.

6. The system of any preceding claim, wherein in the refining of the external control point location, the location of the external control point is constrained to a second predefined area.

7. A non-transitory computer-readable medium storing instructions that, when executed by a processor, cause the processor to perform operations comprising:

    a) receiving a first endpoint location data and a second endpoint location data based on a first and second position sensor assembly respectively mounted near a first and a second end of a flexible spline at a distal tip of a catheter;
    b) performing a coarse determination of a location of an external control point of a quadratic Bezier curve for approximating a shape of the flexible spline, wherein the quadratic Bezier curve is defined by a first and second points corresponding to the first and second endpoints locations and by said external control point, said coarse determination being based on a relationship between a known length of the flexible spline and an estimated length of the quadratic Bezier curve;
    c) refining the location of the external control point by:

    (i) computing a refined length of the quadratic Bezier curve associated with the external control point location determined in step b) by numerical integration of said quadratic Bezier curve;
    (ii) adjusting the location of the external control point to minimize a difference between the known catheter spline length and the refined length of the Bezier curve; and

    d) rendering a shape of the flexible spline based on the quadratic Bezier curve defined by the first endpoint location, the refined external control point location determined in step c), and the second endpoint location.

8. The non-transitory computer-readable medium of claim 7, wherein the external control point location is constrained to a predefined path and performing the coarse determination comprises determining the location of the external control point on said predefined path for which the estimated length of the quadratic Bezier curve is equal to the known length of the flexible spline.

9. The non-transitory computer-readable medium of claim 7 or claim 8, wherein the coarse determination of the location of the external control point is computed using a formula $L_s = (2L_c + L_p)/3$ wherein $L_s$ is the known length of the spline, $L_c$ is the length of the chord connecting the first and second endpoint locations and $L_p$ is the length of a control polyline of the Bezier curve formed by the first endpoint location, the external control point location, and the second endpoint location.

10. The non-transitory computer-readable medium of claim 8 or claim 9, wherein the predefined path is a linear path median and normal to the chord connecting the first and second endpoint locations.

11. The non-transitory computer-readable medium of any one of claims 7 to 10, wherein in the coarse determination, the location of the external control point is constrained to a first predefined area.

12. The non-transitory computer-readable medium of any one of claims 7 to 11, wherein in the refining of the external control point location, the location of the external control point is constrained to a second predefined area.

FIG. 1

FIG. 2

$L_{2C}$
412

402
$P_2$

403
$P_C$

405

$L_C$
410

$L_{1C}$
411

$P_1$
401

FIG. 3

FIG. 4A

FIG. 4B

RECEIVE REAL-TIME COORDINATES
OF SPLINE ENDS $P_1$ AND $P_2$ — 502

RECEIVE LENGTH OF THE SPLINE $L_S$ — 504

DEFINE A NORMAL TO A STRAIGHT LINE
CONNECTING THE SPLINE ENDS $P_1$ AND $P_2$ — 506

CALCULATE AN ESTIMATED LOCATION OF A
QUADRATIC BEZIER CURVE CONTROL POINT $P_C$
ALONG THE NORMAL — 508

COMPUTE A LENGTH OF THE
QUADRATIC BEZIER CURVE $L_B$ — 510

512 — IF $|L_S - L_B| < T$

YES → RENDER A SHAPE OF THE SPLINE
AS A BEZIER CURVE BASED ON
$P_1$, $P_2$ AND $P_C$ — 514

NO

516 — IF $L_S > L_B$

YES → SHIFT THE CONTROL POINT $P_C$ ALONG THE
NORMAL AWAY FROM THE STRAIGHT LINE
CONNECTING THE SPLINE ENDS — 518

NO

SHIFT THE CONTROL POINT $P_C$ ALONG THE NORMAL
TOWARD THE STRAIGHT LINE CONNECTING THE SPLINE ENDS — 520

FIG. 5A

RECEIVE A FIRST ENDPOINT LOCATION DATA AND A SECOND ENDPOINT LOCATION DATA BASED ON A FIRST AND SECOND POSITION SENSOR ASSEMBLY RESPECTIVELY MOUNTED NEAR A FIRST AND A SECOND END OF A FLEXIBLE SPLINE AT A DISTAL TIP OF A CATHETER ⌡ 532

PERFORM A COARSE DETERMINATION OF A LOCATION OF A CONTROL POINT OF A QUADRATIC BEZIER CURVE FOR APPROXIMATING A SHAPE OF THE FLEXIBLE SPLINE, BASED ON A RELATIONSHIP BETWEEN A KNOWN LENGTH OF THE FLEXIBLE SPLINE AND AN ESTIMATED LENGTH OF THE QUADRATIC BEZIER CURVE ⌡ 534

D) REFINE THE LOCATION OF THE CONTROL POINT BY:

(i) COMPUTING A REFINED LENGTH OF THE QUADRATIC BEZIER CURVE ASSOCIATED WITH THE CONTROL POINT LOCATION BY NUMERICAL INTEGRATION OF THE QUADRATIC BEZIER CURVE

(ii)   ADJUSTING THE LOCATION OF THE CONTROL POINT TO MINIMIZE A DIFFERENCE BETWEEN THE KNOWN CATHETER SPLINE LENGTH AND THE REFINED LENGTH OF THE BEZIER CURVE

⌡ 536

RENDER A SHAPE OF THE FLEXIBLE SPLINE BASED ON THE QUADRATIC BEZIER CURVE DEFINED BY THE FIRST ENDPOINT LOCATION, THE REFINED CONTROL POINT LOCATION DETERMINED IN STEP (536), AND THE SECOND ENDPOINT LOCATION ⌡ 538

FIG. 5B

Europäisches
Patentamt

European
Patent Office

Office européen
des brevets

# EUROPEAN SEARCH REPORT

**Application Number**

EP 24 21 2460

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2024/037773 A1 (GOVARI ASSAF [IL]) 1 February 2024 (2024-02-01) * abstract * * paragraphs [0054] - [0066], [0073], [0079]; claims 10,11,19-27; figures 1, 3A-D,7 * | 1-12 | INV. A61B5/06 A61B5/00 A61M25/00 G06T11/20 |
| X,D | US 2024/033008 A1 (GOVARI ASSAF [IL] ET AL) 1 February 2024 (2024-02-01) * abstract * * claims 7-14; figures 3A-D * | 1-12 | |
| A | US 2020/397338 A1 (GLINER VADIM [IL] ET AL) 24 December 2020 (2020-12-24) * abstract * * paragraph [0069]; claims 1-10 * | 1-12 | |
| A | SONG SHUANG ET AL: "Real-Time Shape Estimation for Wire-Driven Flexible Robots With Multiple Bending Sections Based on Quadratic B?zier Curves", IEEE SENSORS JOURNAL, IEEE, USA, [Online] vol. 15, no. 11, 1 November 2015 (2015-11-01), pages 6326-6334, XP011668356, ISSN: 1530-437X, DOI: 10.1109/JSEN.2015.2456181 Retrieved from the Internet: URL:https://ieeexplore.ieee.org/stamp/stam p.jsp?tp=&arnumber=7156055> [retrieved on 2015-09-03] * abstract * * sect.III * | 1-12 | |

TECHNICAL FIELDS
SEARCHED (IPC)

A61B
G06T
A61M

-/--

The present search report has been drawn up for all claims

| Place of search | Date of completion of the search | Examiner |
|---|---|---|
| The Hague | 31 March 2025 | Delval, Christophe |

EPO FORM 1503 03.82 (P04C01)

page 1 of 2

Europäisches
Patentamt

European
Patent Office

Office européen
des brevets

## EUROPEAN SEARCH REPORT

**Application Number**

EP 24 21 2460

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A,D | Levien Raph: "How long is that B?zier? \| Raph Levien's blog", , 28 December 2018 (2018-12-28), pages 1-15, XP093265154, Retrieved from the Internet: URL:https://raphlinus.github.io/curves/2018/12/28/bezier-arclength.html [retrieved on 2025-03-28] * the whole document * ----- | 1-12 | |
| | | | **TECHNICAL FIELDS SEARCHED (IPC)** |

The present search report has been drawn up for all claims

| Place of search | Date of completion of the search | Examiner |
|---|---|---|
| The Hague | 31 March 2025 | Delval, Christophe |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

.................................................................................

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 24 21 2460

This annex lists the patent family members relating to the patent documents cited in the above-mentioned European search report.
The members are as contained in the European Patent Office EDP file on
The European Patent Office is in no way liable for these particulars which are merely given for the purpose of information.

31-03-2025

| Patent document cited in search report | Publication date | Patent family member(s) | Publication date |
|---|---|---|---|
| US 2024037773 A1 | 01-02-2024 | CN 117442208 A | 26-01-2024 |
| | | EP 4311485 A1 | 31-01-2024 |
| | | IL 304561 A | 01-02-2024 |
| | | JP 2024016833 A | 07-02-2024 |
| | | US 2024037773 A1 | 01-02-2024 |
| US 2024033008 A1 | 01-02-2024 | CN 117442207 A | 26-01-2024 |
| | | EP 4311484 A1 | 31-01-2024 |
| | | IL 304562 A | 01-02-2024 |
| | | JP 2024016832 A | 07-02-2024 |
| | | US 2024033008 A1 | 01-02-2024 |
| US 2020397338 A1 | 24-12-2020 | CN 112107365 A | 22-12-2020 |
| | | EP 3753487 A1 | 23-12-2020 |
| | | JP 2021000446 A | 07-01-2021 |
| | | US 2020397338 A1 | 24-12-2020 |

EPO FORM P0459

For more details about this annex : see Official Journal of the European Patent Office, No. 12/82

## REFERENCES CITED IN THE DESCRIPTION

**Patent documents cited in the description**

- US 874224

- US 874224 A

**Non-patent literature cited in the description**

- **RAPH LEVIEN**. How long is that Bézier. *World Wide Web*, https://raphlinus.github.io/curves/2018/12/28/-bezier-arclength.html